# EUROPEAN PATENT APPLICATION

(11) **EP 4 847 010 A2**
(43) Date of publication of application: **02.12.2026**
(21) Application number: 26199667.2
(22) Date of filing: 19.08.2026
(51) Int. Cl.: A61K 8/49, A61K 8/34, A61K 8/368, A61K 8/9789, A61Q 5/02, A61Q 19/00

(54) **SYNERGISTIC ANTIOXIDANT COMPOSITION**

(30) Priority: 20.05.2026 US 202664070505 P
(71) Applicant: The Procter & Gamble Company, Cincinnati, OH 45202 (US)
(72) Inventor: KNOWLES, Sonja Lee, Cincinnati, 45202 (US); LAUGHLIN II, Leo Timothy, Cincinnati, 45202 (US); JOHNSON, Eric Scott, Cincinnati, 45202 (US); PEARSON, Kathleen, Cincinnati, 45202 (US); LOCKER, Kathryn Clare Sullivan, Cincinnati, 45202 (US)
(74) Representative: P&G Patent Germany

(57) **Abstract**

A personal care composition comprising a) rosemary leaf extract; b) a 2-pyridinol-N-oxide material wherein at a level of 0.001% or greater of a and at a level of 0.0025% or greater of b there is a synergistic anti-antioxidant/cellular energetic activity. A personal care composition comprising: a) rosemary leaf extract; b) a 2-pyridinol-N-oxide material; and c) salicylic acid wherein at a level of 0.0075% or greater of a and at a level of 0.0025% or greater of b and at a level of 0.5% or greater of c there is a synergistic anti-antioxidant/cellular energetic activity. A personal care composition comprising: a) rosemary leaf extract; b) a 2-pyridinol-N-oxide material; and c) glycerin wherein at a level of 0.0075% or greater of a and at a level of 0.0025% or greater of b and at a level of 0.001 % or greater of c there is a synergistic anti-antioxidant/cellular energetic activity.

## Description

### FIELD

The present disclosure relates to a personal care composition comprising rosemary leaf extract and piroctone olamine, or rosemary and piroctone olamine and salicylic acid, or rosemary and piroctone olamine and glycerin wherein the personal care composition provides a synergistic antioxidant activity.

### BACKGROUND

The skin is a complex organ in which the overall condition is directly impacted by the oxidative balance. There are many endogenous and exogenous sources of oxidizing species which the skin attempts to counteract with a series of endogenous antioxidant enzymes (such as superoxide dismutase). When the level of oxidizing species is greater than can be counteracted by the skin, the imbalance is called oxidative stress. There are numerous detrimental effects of oxidative stress, such as oxidized lipids and proteins that lead to compromised skin function, such as decreased barrier effectiveness. This can lead to skin dryness, excessive flaking, itch, and hair loss.

The sources of oxidative stress include those of exogenous origin such as pollution (organic and metals), cigarette smoke, ultraviolet light and certain product exposures. Sources of endogenous reactive oxygen species (ROS) include the effects of aging as well as those originating from microbial metabolism. The scalp is an especially susceptible portion of the skin. Malassezia fungi are a known source of ROS. Ultraviolet light exposure is also difficult to avoid on the scalp.

Antioxidants are materials that can supplement the body's ability to defend against ROS. These beneficial agents can be delivered orally or topically. For skin benefits, direct topical delivery is desired.

There are a number of cosmetic factors known to enhance a predisposition to suffering dandruff symptoms. For example, impaired barrier function and resulting dryness make the skin more susceptible to irritants; thus cosmetic hydrating agents can enhance scalp resilience. Likewise, oxidative stress on the surface of the skin contributes to a susceptibility to dandruff and reduction of surface oxidative stress also helps improve resilience. Piroctone olamine is an example of a class of materials called hydroxypyridones, which have a strong affinity to chelate iron. Iron has a fundamental role in oxidative stress via Fenton and Haber-Weiss reactions which can be prevented by iron chelation.

Between increasing environmental ROS sources and decreasing ability to counteract them in an aging population, there is a need for more effective topically delivered antioxidant materials. While there is a wide range of individual antioxidants available, unique combinations have the potential to result in synergistic activities, wherein the delivered activity is more than the sum of individual components. Synergies are difficult to predict and/or identify so finding them is generally an "unexpected" or "surprising" finding. Such combinations are especially useful to supplement the body's native antioxidant activity as we generally get more benefit with less material required.

There is a need to deliver hair retention/fall as well as scalp health benefits from shampoos, conditioners, and leave on treatments. It has been found that botanical extracts can act as anti-oxidants and thus deliver scalp health and hair fall benefits Scalp condition improvement with botanical extracts possessing chemical and biological antioxidant activity. There may be varying actionability constraints amongst different geographies between which "chemical" technologies that can be practiced and combined with the growing consumer desire to achieve "greener" technology solutions in consumer products. The desire is to achieve formulation flexibility along with a greener product lineup AND continue to deliver exceptional efficacy by integrating botanical extracts.

It has been found that rosemary extract is strong in both chemical anti-oxidancy (ORAC) and in biological anti-oxidancy (nrf-2 activation), so it's in vitro profile compares quite favorably with traditional chemistries. Rosemary extract is a renewable botanical so is "green". It also found that rosemary extract may deliver scalp and hair benefits on it's own.

As stated above, Rosemary extract has the in vitro anti-oxidant pedigree and delivers in consumer studies. The surprising discovery is that rosemary extract and piroctone olamine combinations possess synergistic activities in the in vitro anti-oxidant assay, the HOOH-ATP assay. The HOOH-ATP assay measures how much cellular energy a skin cell has when cotreated with a technology while undergoing stress from hydrogen peroxide (to mimic reactive oxygen stress found in skin and hair follicles).

### SUMMARY

A personal care composition comprising a) rosemary leaf extract; b) a 2-pyridinol-N-oxide material wherein at a level of 0.001% or greater of a and at a level of 0.0025% or greater of b there is a synergistic anti-antioxidant/cellular energetic activity. A personal care composition comprising: a) rosemary leaf extract; b) a 2-pyridinol-N-oxide material; and c) salicylic acid wherein at a level of 0.0075% or greater of a and at a level of 0.0025% or greater of b and at a level of 0.5% or greater of c there is a synergistic anti-antioxidant/cellular energetic activity.

A personal care composition comprising: a) rosemary leaf extract; b) a 2-pyridinol-N-oxide material; and c) glycerin wherein at a level of 0.0075% or greater of a and at a level of 0.0025% or greater of b and at a level of 0.001% or greater of c there is a synergistic anti-antioxidant/cellular energetic activity.

### BRIEF DESCRIPTION OF THE DRAWINGS

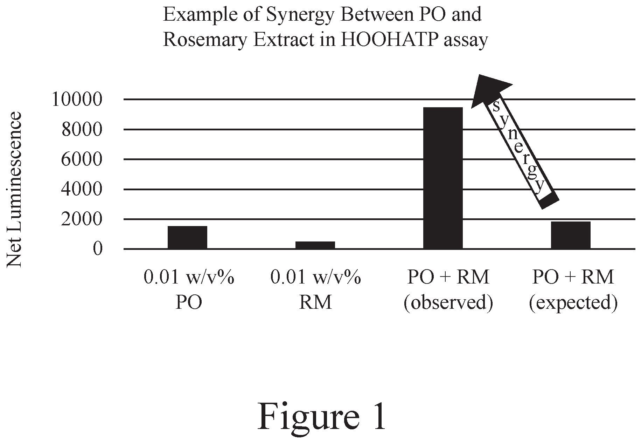

Figure 1 is a Peroxide-Induced Keratinocyte Stress assay demonstrating a synergistic level of activity for a dyad (piroctone olamine and rosemary leaf extract) over the calculated sum of individual observations and is a graphical representation of the synergy data representing the ingredient levels of a personal care composition.

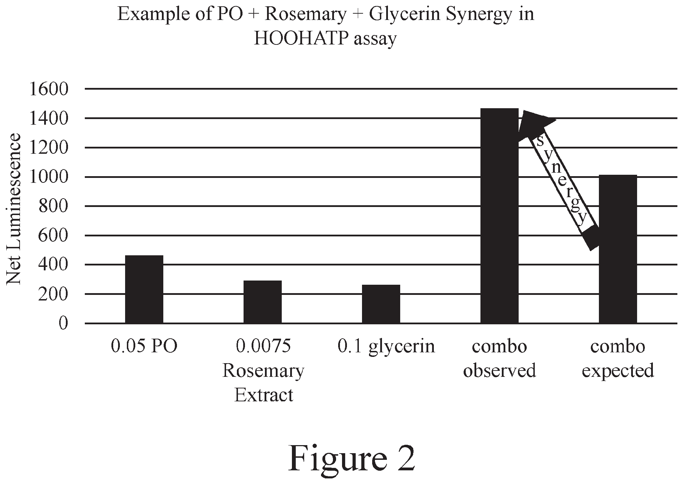

Figure 2 is a Peroxide-Induced Keratinocyte Stress assay demonstrating a synergistic level of activity for a tirad (rosemary leaf extract and piroctone olamine and glycerin) over the calculated sum of individual observations and is a graphical representation of the synergy data representing the ingredient levels of a personal care composition.

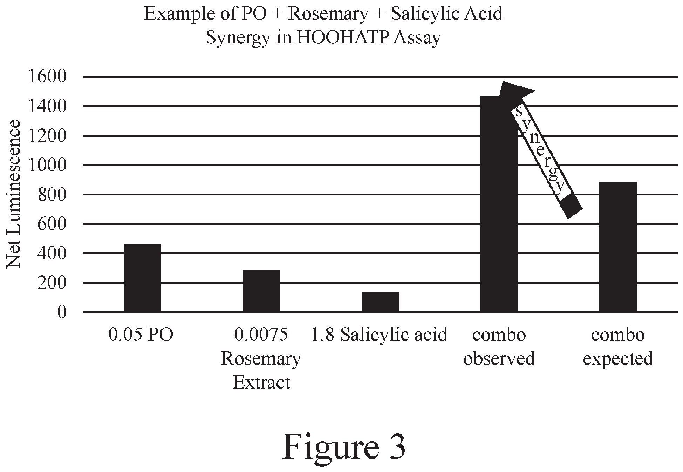

Figure 3 is a Peroxide-Induced Keratinocyte Stress assay demonstrating a synergistic level of activity for a tirad (rosemary leaf extract and piroctone olamine and salicylic acid) over the calculated sum of individual observations and is a graphical representation of the synergy data representing the ingredient levels of a personal care composition.

### DETAILED DESCRIPTION

All percentages and ratios used herein are by weight of the total composition, unless otherwise designated. All measurements are understood to be made at ambient conditions, where "ambient conditions" means conditions at about 25°C, under about one atmosphere of pressure, and at about 50% relative humidity, unless otherwise designated. All numeric ranges are inclusive of narrower ranges; delineated upper and lower range limits are combinable to create further ranges not explicitly delineated.

The personal care composition can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

"Apply" or "application," as used in reference to a composition, means to apply or spread the compositions onto keratinous tissue such as the hair.

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

While the specification concludes with claims particularly pointing out and distinctly claiming the present disclosure, it is believed it will be better understood from the following description.

As used herein, the term "fluid" includes liquids and gels.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of".

As used herein, "mixtures" is meant to include a simple combination of materials and any compounds that may result from their combination.

As used herein, "molecular weight" or "Molecular weight" refers to the weight average molecular weight unless otherwise stated. Molecular weight is measured using industry standard method, gel permeation chromatography ("GPC").

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the personal care composition.

The term "coacervate" as used herein, means the complex which forms between surfactant and polymer that may either be soluble or insoluble in the neat composition, typically forming an insoluble complex in the neat composition, and which may become less soluble upon dilution and thus yielding an increase in its level of phase separation or precipitate in solution.

The term "isotropic" as used herein, means a particular phase structure of coacervate wherein the structure is "identical along any three orthogonal directions in space, and is therefore dark or 'nonbirefringent' when viewed between crossed polarized light. (One direction is 'orthogonal' to another if the vector component of the first, in the direction of the second, is zero.) (Laughlin, R. G. (1994). "The Aqueous Phase Behavior of Surfactants," 182, 8.2).

As used herein, "personal care compositions" includes products such as shampoos, shower gels, liquid hand cleansers, hair colorants, facial cleansers, beard washes, intimate washes, and other surfactant-based liquid compositions intended to clean scalp and bodily hair and skin.

As used herein, the terms "include," "includes," and "including," are meant to be non-limiting and are understood to mean "comprise," "comprises," and "comprising," respectively.

All percentages, parts and ratios are based upon the total weight of the compositions of the present disclosure, unless otherwise specified. All such weights as they pertain to listed ingredients are based on the active level and, therefore, do not include carriers or by-products that may be included in commercially available materials.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Rosemary

Rosmarinus officinalis L., commonly known as rosemary, is a Mediterranean herb from which standardized extracts are derived. These extracts contain a rich profile of bioactive compounds, including rosmarinic acid, caffeic acid, and carnosic acid, along with essential oils. Traditionally used in culinary and medicinal practices, rosemary leaf standardized extract is primarily recognized for its potential in cognitive enhancement, antioxidant support, antiinflammatory properties, and benefits for skin health.

*Salvia rosmarinus*, synonym *Rosmarinus officinalis*, commonly known as rosemary, is a shrub with fragrant, evergreen, needle-like leaves and purple or sometimes white, pink, or blue flowers. It is a member of the mint family, Lamiaceae. The species is native to the Mediterranean region.

Rosemary leaf extract represents non-volatile chemistry. In this instance, it is a water extract that contains a blend of various natural chemical classes, including alkaloids, terpenes, flavonoids, polyphenols, and polymers, with molecular weights typically ranging from 150 to over 2000. In contrast, rosemary essential oil is characterized by its volatile chemistry, primarily composed of volatile terpenes, mainly monoterpenes with some diterpenes, all with molecular weights under 300. This essential oil is typically obtained by steaming the rosemary plant and collecting the oil that is vaporized out of the plant.

The personal care composition may comprise actives in the form of refined plant extracts containing the actives. For example, the personal cleansing composition may comprise Rosmarinic acid that is added to the formula through a refined plant extract. Exemplary refined plant extracts can be sourced from a variety of botanicals.

For rosemary leaf extract, it is noted that there may be personal care compositions that may list rosemary extract vs. rosemary leaf extract as an ingredient. The personal care composition may have a rosemary leaf extract, which is different from an extract from rosemary that is the essential oil.

The personal care composition may comprise from about 0.001% to 3% of rosemary leaf extract, from about 0.001% to 2% of rosemary leaf extract, and from about 0.001% to 1% of rosemary leaf extract.

### 2-Pyridinol-N-Oxide Materials

2-Pyridinol-N-oxide materials suitable for use in this personal care composition include a substituted or unsubstituted 2-pyridinol-N-oxide material or a salt thereof. Included are tautomers of this material, e.g., 1-hydroxy-2(1H)-pyridinone. The substituted or unsubstituted 2-pyridinol-N-oxide material and its corresponding tautomeric form, 1-hydroxy-2(1H)-pyridinone, are shown below: 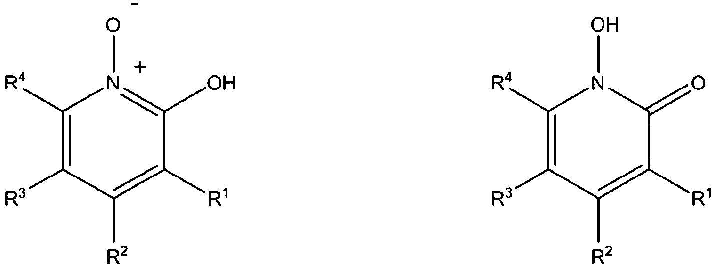 where R¹, R², R³, R⁴ groups are independently selected from the group consisting of H, Cl, Br, I, F, NO, NO₂, and (CH₂)ₙG, where each G is independently selected from the group consisting of (O)ₘSO₃M³, (O)ₘCO₂M³, (O)ₘC(O)(R⁵), (O)ₘC(O)N(R⁵R⁶), (O)ₘCN, (O)ₘ(R⁵), and N(R⁵R⁶), where m is 0 or 1, n is an integer from 0 to 4, R⁵ and R⁶ are independently selected from the group consisting of H and a substituted or unsubstituted C₁-C₁₂ organic group, and M³ is selected from the group consisting of H, a substituted or unsubstituted C₁-C₁₂ organic group, ⁺N(R⁷R⁸R⁹R¹⁰), and 1/q M' ^{q+} where M' is selected from the group consisting of an alkali metal of charge q and an alkaline earth metal of charge q, where R7, R8, R9, and R10 are independently selected from the group consisting of H and a substituted or unsubstituted C₁-C₁₂ organic group, and where any pair of vicinal groups, R¹ and R², R² and R³, R³ and R⁴ may be taken together to form another five- or six-membered aromatic or aliphatic ring optionally substituted with one or more groups selected from the group consisting of Cl, Br, I, F, NO, NO₂, CN, (CH₂)ₙG, and mixtures thereof. Suitable organic groups include (C₁-C₁₂)alkyl, (C₂-C₁₂)alkenyl, and (C₂-C₁₂)alkynyl. The organic group may optionally be substituted and suitable substituent groups include a hydroxyl group, a carboxyl group, and an amino group. 2-pyridinol-N-oxide is also known, for example, as 2-hydroxypyridine-N-oxide, 2-pyridinol-1-oxide, or 2-hydroxypyridine-1-oxide.

In certain aspects, the 2-pyridinol-N-oxide material is a 2-pyridinol-N-oxide material or tautomer thereof according to the formula(s) above, where R¹, R², R³, R⁴ are independently selected from the group consisting of H, Cl, and (CH₂)ₙG, where G is independently selected from the group consisting of (O)ₘSO₃M³, (O)ₘCO₂M³, (O)ₘC(O)(R⁵), (O)ₘCN, and (O)ₘ(R⁵), where m is 0 or 1. In other aspects, the 2-pyridinol-N-oxide material is a 2-pyridinol-N-oxide material according to the formula above, where R¹, R², R³, R⁴ are independently selected from the group consisting of H, SO₃M³, and CO₂M³. In still other aspects, R¹, R², R³, R⁴ are independently selected from the group consisting of H, SO₃M³, and CO₂M³, where no more than one R¹, R², R³, R⁴is SO₃M³ or CO₂M³.

In certain aspects, the 2-pyridinol-N-oxide material is the salt of a substituted or unsubstituted 2-pyridinol-N-oxide material. In these aspects, the hydrogen of the hydroxyl group of the 2-pyridinol-N-oxide material may be substituted with a suitable charge-balancing cation. In these aspects, non-limiting examples of the hydrogen-substituting cation include Na⁺, Li⁺, K⁺, ½ Mg²⁺, or ½ Ca²⁺, substituted ammonium, such as C₁-C₆ alkanolammnonium, mono-ethanolamine (MEA), tri-ethanolamine (TEA), di-ethanolamine (DEA), or any mixture thereof. In some aspects, in solution, the cation may be dissociated from the 2-pyridinol-N-oxide or the 1-hydroxy-2(1H)-pyridinone anion.

In certain aspects, the 2-pyridinol-N-oxide material is of a substituted or unsubstituted 2-pyridinol-N-oxide material. Salts for use herein include those formed from the polyvalent metals barium, bismuth, strontium, copper, zinc, cadmium, zirconium and mixtures thereof.

In some aspects, the 2-pyridinol-N-oxide material is selected from the group consisting of: 6-hydroxy-3-pyridinesulfonic acid, 1-oxide (CAS 191672-18-1); 2-hydroxypyridine-1-oxide (CAS 13161-30-3); 2-hydroxy-4-pyridinecarboxylic acid, 1-oxide (CAS 13602-64-7); 5-ethoxy-2-pyridinol, 2-acetate, 1-oxide (CAS 51984-49-7); 1-(3-hydroxy-2-oxido-4-isoquinolinyl)-ethanone (CAS 65417-65-4); 6-hydroxy-3-pyridinecarboxylic acid, 1-oxide (CAS 90037-89-1); 2-methoxy-4-quinolinecarbonitrile, 1-oxide (CAS 379722-76-6); 2-pyridinecarboxylic acid, 6-hydroxy-, 1-oxide (CAS 1094194-45-2); 3-pyridinecarboxylic acid, 2-hydroxy-, 1-oxide (CAS 408538-43-2); 2-pyridinol, 3-nitro-, 1-oxide (CAS 282102-08-3); 3-pyridinepropanenitrile, 2-hydroxy-, 1-oxide (193605-60-6); 3-pyridineethanol, 2-hydroxy-, 3-acetate, 1-oxide (CAS 193605-56-0); 2-pyridinol, 4-bromo-, 1-oxide (CAS 170875-41-9); 2-pyridinol, 4,6-dibromo-, 2-acetate, 1-oxide (CAS 170875-40-8); 2-pyridinol, 4,6-dibromo, 1-oxide (CAS 170875-38-4); 2-pyridinol, 4-(2-aminoethyl)-, 1-oxide (CAS 154403-93-7); 2-pyridinol, 5-(2-aminoethyl)-, 1-oxide (CAS 154403-92-6); 3-pyridinepropanoic acid, α-amino-6-hydroxy-, 1-oxide (CAS 134419-61-7); 2-pyridinol, 3,5-dimethyl, 1-oxide (CAS 102074-62-4); 2-pyridinol, 3-methyl-, 1-oxide (CAS 99969-07-0); 2-pyridinol, 3,5-dinitro, 1-oxide (CAS 98136-47-1); 2-pyridinol, 3,5-dibromo-, 1-oxide (CAS 98136-29-9); 2-pyridinol, 4-methyl-6-(2-methylpropyl)-, 1-oxide (CAS 91408-77-4); 2-pyridinol, 3-bromo-4,6-dimethyl-, 1-oxide (CAS 91408-76-3); 2-pyridinol, 4,5,6-trimethyl-, 1-oxide (CAS 91408-75-2); 2-pyridinol, 6-heptyl-4-methyl-, 1-oxide (CAS 91408-73-0); 2-pyridinol, 6-(cyclohexylmethyl)-4-methyl-, 1-oxide (CAS 91408-72-9); 2-pyridinol, 6-bromo-, 1-oxide (CAS 89284-00-4); 2-pyridinol, 5-bromo-, 1-oxide (CAS 89283-99-8); 2-pyridinol, 3,5-dichloro-4,6-difluoro-, 1-oxide (CAS 33693-37-7); 2-pyridinol, 3,4,5,6-tetrachloro-, 1-oxide (CAS 32835-63-5); 2-pyridinol, 6-methyl-, 1-oxide (CAS 14420-62-3); 2-pyridinol, 5-nitro-, 1-oxide (CAS 14396-03-3); 2-pyridinol, 4-methyl-5-nitro-, 1-oxide (CAS 13602-77-2); 2-pyridinol, 4-chloro-5-nitro-, 1-oxide (CAS 13602-73-8); 2-pyridinol, 4-chloro-, 1-oxide (CAS 13602-65-8); 2-pyridinol, 4-nitro-, 1-oxide (CAS 13602-63-6); and 2-pyridinol, 4-methyl-, 1-oxide (CAS 1952-64-3), and mixtures thereof. These materials are commercially available from, for example, Sigma-Aldrich (St. Louis, MO) and/or Aces Pharma (Branford, CT).

In certain aspects, the 2-pyridinol-N-oxide material is a 2-pyridinol-N-oxide material selected from the group consisting of: 2-hydroxypyridine-1-oxide; 3-pyridinecarboxylic acid, 2-hydroxy-, 1-oxide; 6-hydroxy-3-pyridinecarboxylic acid, 1-oxide; 2-hydroxy-4-pyridinecarboxylic acid, 1-oxide; 2-pyridinecarboxylic acid, 6-hydroxy-, 1-oxide; 6-hydroxy-3-pyridinesulfonic acid, 1-oxide; and mixtures thereof.

In certain aspects, the 2-pyridinol-N-oxide material is a 1-Hydroxy-2(1H)-pyridinone material selected from the group consisting of: 1-Hydroxy-2(1H)-pyridinone (CAS 822-89-9); 1,6-dihydro-1-hydroxy-6-oxo-3-Pyridinecarboxylic acid (CAS 677763-18-7); 1,2-dihydro-1-hydroxy-2-oxo-4-Pyridinecarboxylic acid (CAS 119736-22-0); 1,6-dihydro-1-hydroxy-6-oxo-2-Pyridinecarboxylic acid (CAS 94781-89-2); 1-hydroxy-4-methyl-6-(2,4,4-trimethylpentyl)-2(1H)-Pyridinone (CAS 50650-76-5); 6-(cyclohexylmethyl)-1-hydroxy-4-methyl-2(1H)-Pyridinone (CAS 29342-10-7); 1-hydroxy-4,6-dimethyl-2(1H)-Pyridinone (CAS 29342-02-7); 1-Hydroxy-4-methyl-6-(2,4,4-trimethylpentyl)-2-pyridone monoethanolamine (CAS 68890-66-4); 1-hydroxy-6-(octyloxy)-2(1H)-Pyridinone (CAS 162912-64-3); 1-Hydroxy-4-methyl-6-cyclohexyl-2-pyridinone ethanolamine salt (CAS 41621-49-2); 1-Hydroxy-4-methyl-6-cyclohexyl-2-pyridinone (CAS 29342-05-0); 6-ethoxy-1,2-dihydro-1-hydroxy-2-oxo-4-Pyridinecarboxylic acid,methyl ester (CAS 36979-78-9); 1-hydroxy-5-nitro -2(1H)-Pyridinone (CAS 45939-70-6); and mixtures thereof. These materials are commercially available from, for example, Sigma-Aldrich (St. Louis, MO), Princeton Building Blocks (Monmouth Junction, NJ), 3B Scientific Corporation (Libertyville, IL), SynFine Research (Richmond Hill, ON), Ryan Scientific, Inc. (Mt. Pleasant, SC), and/or Aces Pharma (Branford, CT).

In certain aspects, the 2-pyridinol-N-oxide material is a 2-pyridinol-N-oxide material or tautomer thereof according to the formula(s) below: 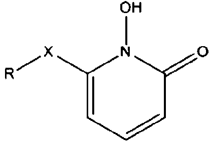 where X is an oxygen or sulfur moiety and R is a substituted or unsubstituted hydrocarbon group having between 1 and 20 carbon atoms. Materials of this class can be synthesized following the procedure disclosed in US 5,675,013.

In certain aspects, the 2-pyridinol-N-oxide material is a 2-pyridinol-N-oxide material or tautomer thereof according to the formula(s) below: 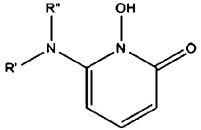 wherein R' and R" are independently either hydrogen or a substituted or unsubstituted hydrocarbon group having between 1 and 20 carbon atoms. Materials of this class can be synthesized following the procedure disclosed in US 5,675,013. In certain aspects, the 2-pyridinol-N-oxide material is 1-Hydroxy-4-methyl-6-(2,4,4-trimethylpentyl)-2-pyridone monoethanolamine salt. The personal care composition may comprise a hydroxy pyridone. Further, the personal care composition may comprise a hydroxy pyridone which is piroctone olamine. Nonlimiting examples of hydroxy pyridones may include octopirox (piroctone olamine), ciclopirox, and rilopirox.

The personal care composition may contain from about 0.1% to about 10% of a hydroxy pyridone. The personal care composition may contain from about 0.3% to about 3% of a hydroxy pyridone. The personal care composition may contain from about 0.5% to about 2% of a hydroxy pyridone.

### Salicylic Acid

Salicylic acid suitable for use in this personal care composition include salicylic acid or a salt thereof. In the personal care composition such salicylic acid salts may include alkali metal salts, such as sodium and potassium; alkaline earth metal salts, such as calcium and magnesium; ammonium salts; and trialkylammonium salts, such as trimethylammonium and triethylammonium. The personal care composition may include capryloyl salicylic acid, methyl salicylate, acetyl salicylate, sodium salicylate, choline salicylate, methyl salicylic acid, acetyl salicylic acid, amino salicylic acid, salicylsalicylic acid, menthyl salicylate, phenyl salicylate, amyl salicylate, octyl salicylate, benzyl salicylate, glyceryl salicylate, salicin, salicylaldehyde, salicortin, 2'-O-acetylsalicortin, (-)-tremulacin, saligenin, diflunisal, fendosal, butyloctyl salicylate, C12-15 alkyl salicylate, hexyldodecyl salicylate, isocetyl salicylate, isodecyl salicylate, monoethanolamine salicylate, ethylhexyl salicylate, myristyl salicylate, trolamine salicylate, and tridecyl salicylate The personal care composition may also include salicylates in the form of natural products such as oil of wintergreen and willow bark extract.

The personal care composition may contain from about 0.1% to about 10% of a salicylic acid; the hair care composition may contain from about 0.3% to about 3% of a salicylic acid; the hair care composition may contain from about 0.5% to about 2% of salicylic acid.

### Glycerin

Glycerin may be present from about 0.05% to about 30%, from about 0.1% to about 15%, from about 0.5% to about 10%; from about 0.5% to 5%, from about 0.5% to 2%.

The glycerin may be any glycerin that can be used in personal care compositions, and may include synthetic glycerin produced by a synthetic method and natural glycerin produced by saponifying naturally-derived fats and oils.

### DETERSIVE SURFACTANT

The personal care composition may be present in the form of a shampoo, conditioner, or leave on treatment. The shampoo composition may comprise one or more detersive surfactants, which provides cleaning performance to the composition. The one or more detersive surfactants in turn may comprise an anionic surfactant, amphoteric or zwitterionic surfactants, or mixtures thereof. Various examples and descriptions of detersive surfactants are set forth in U.S. Patent No. 6,649,155; U.S. Patent Application Publication No. 2008/0317698; and U.S. Patent Application Publication No. 2008/0206355, which are incorporated herein by reference in their entirety.

The concentration of the detersive surfactant component in the shampoo composition should be sufficient to provide the desired cleaning and lather performance, and generally ranges from about 2 wt% to about 50 wt%, from about 5 wt% to about 30 wt%, from about 8 wt% to about 25 wt%, from about 10 wt% to about 20 wt%, about 5 wt%, about 10 wt%, about 12 wt%, about 15 wt%, about 17 wt%, about 18 wt%, or about 20 wt%.

Anionic surfactants suitable for use in the compositions may be alkyl and alkyl ether sulfates. Other suitable anionic surfactants are the water soluble salts of organic, sulfuric acid reaction products. Still other suitable anionic surfactants are the reaction products of fatty acids esterified with isethionic acid and neutralized with sodium hydroxide. -Other similar anionic surfactants are described in U.S. Patent Nos. 2,486,921; 2,486,922; and 2,396,278, which are incorporated herein by reference in their entirety.

Exemplary anionic surfactants for use in the shampoo composition include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate, acyl glycinates, acyl sarcosinates, acyl glutamates, acyl alaninates, sulfosuccinates, isethionates, sulfonates, sulfoacetates, glucose carboxylates, alkyl ether carboxylates, acyl taurates, sodium, ammonium or potassium salts of these, alpha-olefin sulfonates; sodium, ammonium or potassium salts of isethionates; sodium, ammonium or potassium salts of sulfonates; sodium, ammonium or potassium salts of ether sulfonates; sodium, ammonium or potassium salts of sulfosuccinates; sodium, ammonium or potassium salts of sulfoacetates; sodium, ammonium or potassium salts of glycinates; sodium, ammonium or potassium salts of sarcosinates; sodium, ammonium or potassium salts of glutamates; sodium, ammonium or potassium salts of alaninates; sodium, ammonium or potassium salts of carboxylates; sodium, ammonium or potassium salts of taurates; sodium, ammonium or potassium salts of phosphate esters and combinations thereof. The anionic surfactant may be sodium lauryl sulfate or sodium laureth sulfate.

Suitable amphoteric or zwitterionic surfactants for use in the shampoo composition herein include those which are known for use in shampoo or other personal care cleansing compositions. Concentrations of such amphoteric surfactants range from about 0.5 wt% to about 20 wt%, and from about 1 wt% to about 10 wt%. Non limiting examples of suitable zwitterionic or amphoteric surfactants are described in U.S. Patent Nos. 5,104,646 and 5,106,609, which are incorporated herein by reference in their entirety.

Amphoteric detersive surfactants suitable for use in the shampoo composition include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic group such as carboxy, sulfonate, sulfate, phosphate, or phosphonate. Exemplary amphoteric detersive surfactants for use in the present shampoo composition include cocoamphoacetate, cocoamphodiacetate, lauroamphoacetate, lauroamphodiacetate, and mixtures thereof.

Zwitterionic detersive surfactants suitable for use in the shampoo composition include those surfactants broadly described as derivatives of aliphatic quaternaryammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. Further, zwitterionics such as betaines may be selected.

Non limiting examples of other anionic, zwitterionic, amphoteric or optional additional surfactants suitable for use in the shampoo composition are described in McCutcheon's, Emulsifiers and Detergents, 1989 Annual, published by M. C. Publishing Co., and U.S. Patent Nos. 3,929,678, 2,658,072; 2,438,091; 2,528,378, which are incorporated herein by reference in their entirety.

The shampoo composition may also comprise a shampoo gel matrix, an aqueous carrier, and other additional ingredients described herein.

### AQUEOUS CARRIER

The shampoo composition comprises a first aqueous carrier. Accordingly, the formulations of the shampoo composition can be in the form of pourable liquids (under ambient conditions). Such compositions will therefore typically comprise a first aqueous carrier, which is present at a level of at least 20 wt%, from about 20 wt% to about 95 wt%, or from about 60 wt% to about 85 wt%. The first aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The first aqueous carriers useful in the shampoo composition include water and water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, hexylene glycol, glycerin, and propane diol.

A conditioner gel matrix of a conditioner composition includes a second aqueous carrier. Accordingly, the formulations of the conditioner composition can be in the form of pourable liquids (under ambient conditions). Such compositions will therefore typically comprise a second aqueous carrier, which is present at a level of from about 20 wt% to about 95 wt%, or from about 60 wt% to about 85 wt%. The second aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The second aqueous carriers useful in the conditioner composition include water and water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, hexylene glycol, glycerin, and propane diol.

### ADDITIONAL COMPONENTS

The shampoo composition, conditioner compositions, and/or leave-on treatments described herein may optionally comprise one or more additional components known for use in personal care products, provided that the additional components are physically and chemically compatible with the essential components described herein, or do not otherwise unduly impair product stability, aesthetics or performance. Such additional components are most typically those described in reference books such as the CTFA Cosmetic Ingredient Handbook, Second Edition, The Cosmetic, Toiletries, and Fragrance Association, Inc. 1988, 1992. Individual concentrations of such additional components may range from about 0.001 wt% to about 10 wt% by weight of the personal care compositions.

Non-limiting examples of additional components for use in the personal care compositions include conditioning agents , natural cationic deposition polymers, synthetic cationic deposition polymers, anti-dandruff agents, particles, suspending agents, paraffinic hydrocarbons, propellants, viscosity modifiers, dyes, non-volatile solvents or diluents (water-soluble and water-insoluble), pearlescent aids, foam boosters, additional surfactants or nonionic cosurfactants, pediculocides, pH adjusting agents, perfumes, preservatives, proteins, skin active agents, sunscreens, UV absorbers, and vitamins.

### 1. Conditioning Agent

The personal care compositions may comprise one or more conditioning agents. Conditioning agents include materials that are used to give a particular conditioning benefit to hair or skin. The conditioning agents useful in the personal care compositions typically comprise a water-insoluble, water-dispersible, non-volatile, liquid that forms emulsified, liquid particles. Suitable conditioning agents for use in the personal care composition are those conditioning agents characterized generally as silicones , organic conditioning oils or combinations thereof, or those conditioning agents which otherwise form liquid, dispersed particles in the aqueous surfactant matrix.

One or more conditioning agents are present from about 0.01 wt% to about 10 wt%, from about 0.1 wt% to about 8 wt%, and from about 0.2 wt% to about 4 wt%, by weight of the composition.

### Silicone Conditioning Agent

The compositions of the personal care composition may contain one or more silicone conditioning agents. Examples of the silicones include dimethicones, dimethiconols, cyclic silicones, methylphenyl polysiloxane, and modified silicones with various functional groups such as amino groups, quaternary ammonium salt groups, aliphatic groups, alcohol groups, carboxylic acid groups, ether groups, epoxy groups, sugar or polysaccharide groups, fluorine-modified alkyl groups, alkoxy groups, or combinations of such groups. Such silicones may be soluble or insoluble in the aqueous (or non-aqueous) product carrier. In the case of insoluble liquid silicones, the polymer can be in an emulsified form with droplet size of about 10 nm to about 30 micrometers

### Organic Conditioning Materials

The conditioning agent of the compositions of the personal care composition may also comprise at least one organic conditioning material such as oil or wax, either alone or in combination with other conditioning agents, such as the silicones described above. The organic material can be nonpolymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to about 2,000,000 including those with CTFA names PEG-20 200, PEG-400, PEG-600, PEG-1000, PEG-2M, PEG-7M, PEG-14M, PEG-45M and mixtures thereof.

### Thickening Polymers

The personal care composition may comprise a thickening polymer to increase the viscosity of the composition. Suitable thickening polymers can be used. The personal care composition may comprise from about 0.25% to about 10% of a thickening polymer, from about 0.5% to about 8% of a thickening polymer, from about 1.0% to about 5% of a thickening polymer, and from about 1% to about 4% of a thickening polymer. The thickening polymer modifier may be a polyacrylate, polyacrylamide thickeners. The thickening polymer may be an anionic thickening polymer.

The personal care composition may comprise thickening polymers that are homopolymers based on acrylic acid, methacrylic acid or other related derivatives, non-limiting examples include polyacrylate, polymethacrylate, polyethylacrylate, and polyacrylamide.

The thickening polymers may be alkali swellable and hydrophobically-modified alkali swellable acrylic copolymers or methacrylate copolymers, non-limiting examples include acrylic acid/acrylonitrogens copolymer, acrylates/steareth-20 itaconate copolymer, acrylates/ceteth-20 itaconate copolymer, Acrylates/Aminoacrylates/C10-30 Alkyl PEG-20 Itaconate Copolymer, acrylates/aminoacrylates copolymer, acrylates/steareth-20 methacrylate copolymer, acrylates/beheneth-25 methacrylate copolymer, acrylates/steareth-20 methacrylate crosspolymer, acrylates/beheneth-25 methacrylate/HEMA crosspolymer, acrylates/vinyl neodecanoate crosspolymer, acrylates/vinyl isodecanoate crosspolymer, Acrylates/Palmeth-25 Acrylate Copolymer, Acrylic Acid/Acrylamidomethyl Propane Sulfonic Acid Copolymer, and acrylates/C10-C30 alkyl acrylate crosspolymer.

The thickening polymers may be soluble crosslinked acrylic polymers, a non-limiting example includes carbomers.

The thickening polymers may be an associative polymeric thickeners, non-limiting examples include: hydrophobically modified, alkali swellable emulsions, non-limiting examples include hydrophobically modified polypolyacrylates; hydrophobically modified polyacrylic acids, and hydrophobically modified polyacrylamides; hydrophobically modified polyethers wherein these materials may have a hydrophobe that can be selected from cetyl, stearyl, oleayl, and combinations thereof.

Non-limiting examples of thickening polymers include acrylamide/ammonium acrylate copolymer (and) polyisobutene (and) polysorbate 20; acrylamide/sodium acryloyldimethyl taurate copolymer/isohexadecane/polysorbate 80, ammonium acryloyldimethyltaurate/VP copolymer, Sodium Acrylate/Sodium Acryloyldimethyl Taurate Copolymer, acrylates copolymer, Acrylates Crosspolymer-4, Acrylates Crosspolymer-3, acrylates/beheneth-25 methacrylate copolymer, acrylates/C10-C30 alkyl acrylate crosspolymer, acrylates/steareth-20 itaconate copolymer, ammonium polyacrylate/Isohexadecane/PEG-40 castor oil; carbomer, sodium carbomer, crosslinked polyvinylpyrrolidone (PVP), polyacrylamide/C13-14 isoparaffin/laureth-7, polyacrylate 13/polyisobutene/polysorbate 20, polyacrylate crosspolymer-6, polyamide-3, polyquaternium-37 (and) hydrogenated polydecene (and) trideceth-6, Acrylamide/Sodium Acryloyldimethyltaurate/Acrylic Acid Copolymer, sodium acrylate/acryloyldimethyltaurate/dimethylacrylamide, crosspolymer (and) isohexadecane (and) polysorbate 60, sodium polyacrylate. Exemplary commercially-available thickening polymers include ACULYN^{™} 28, ACULYN^{™} 33, ACULYN^{™} 88, ACULYN^{™} 22, ACULYN^{™} Excel, FlexiThix^{™}, Carbopol^{®} Aqua SF-1, Carbopol^{®} ETD 2020, Carbopol^{®} Ultrez 20, Carbopol^{®} Ultrez 21, Carbopol^{®} Ultrez 10, Carbopol^{®} Ultrez 30, Carbopol^{®} 1342, Carbopol^{®} Aqua SF-2 Polymer, Sepigel^{™} 305, Simulgel^{™} 600, Sepimax Zen, Carbopol^{®} SMART 1000, Rheocare^{®} TTA, Rheomer^{®} SC-Plus, STRUCTURE^{®} PLUS, Aristoflex^{®} AVC, Stabylen 30 and combinations thereof.

### Benefit Agents

The personal care composition may further comprise one or more additional benefit agents. The benefit agents comprise a material selected from the group consisting of anti-dandruff agents, anti-fungal agents, anti-itch agents, anti-bacterial agents, anti-microbial agents, moisturization agents, anti-oxidants, vitamins, lipid soluble vitamins, perfumes, brighteners, enzymes, sensates, attractants, dyes, pigments, bleaches, and mixtures thereof.

The personal care compositions may be presented in typical personal care formulations. They may be in the form of solutions, dispersion, emulsions, powders, talcs, encapsulated, spheres, spongers, solid dosage forms, foams, and other delivery mechanisms. The personal care compositions may be hair tonics, leave-on hair products such as treatment, and styling products, rinse-off hair products such as shampoos, and treatment products; and any other form that may be applied to hair.

The personal care compositions are generally prepared by conventional methods such as are known in the art of making the compositions. Such methods typically involve mixing of the ingredients in one or more steps to a relatively uniform state, with or without heating, cooling, application of vacuum, and the like. The compositions are prepared such as to optimize stability (physical stability, chemical stability, photostability) and/or delivery of the active materials. The personal care composition may be in a single phase or a single product, or the personal care composition may be in a separate phases or separate products. If two products are used, the products may be used together, at the same time or sequentially. Sequential use may occur in a short period of time, such as immediately after the use of one product, or it may occur over a period of hours or days.

### Methods

### Peroxide-Induced Keratinocyte Stress

The principle used in this in vitro cellular methodology is oxidatively stress keratinocytes, which depletes their cellular energy (ATP) and then to quantify the ability of various materials to inhibit the negative consequences of the oxidative stress (higher ATP is more effective) and as demonstrated in Figures 1, 2, and 3.

This methodology is further discussed in Assessing bioenergetic function in response to oxidative stress by metabolic profiling. Free Radical Biology and Medicine; Dranka BP, Benavides GA, Diers AR, Giordano S, Zelickson BR, Reily C, Zou L, Chatham JC, Hill BG, Zhang J, Landar A; 2011 Nov 1;51(9):1621-35, incorporated herein by reference, as well as in Proceedings of the National Academy of Sciences of the United States of America; Miyoshi N, Oubrahim H, Chock PB, Stadtman ER... 2006 Feb 7;103(6):1727-31, incorporated herein by reference.

Experimental Methodology: This method demonstrates the synergistic reduction in Adenosine Triphosphate (ATP) depletion caused by Reactive Oxygen Species (ROS) Caused by either binary combination of piroctone olamine and rosemary extract and the ternary combinations of piroctone olamine + rosemary extract+ salicylic acid and piroctone olamine + rosemary extract + glycerin. Hydrogen peroxide is a well-known ROS as is commonly used as a surrogate to analyze the effects of a variety of ROS. In this test, keratinocytes are cultured (passage <8) in T150 flasks using Epi-Life medium (Calcium Free and Phenol Red Free, supplemented with penicillin/streptomycin and keratinocytes growth supplement, Invitrogen cat# MEPICFPRF500) in a CO2 incubator (Forma 51030532, Marietta OH). The keratinocytes are then plated into 96 well plates, 10,000 cells/well, 0.2 ml media. After 24 hours incubation in 37°C in CO2 Incubator, the keratinocytes are treated with 500 uM hydrogen peroxide alone and with a constant dilution of (for example) 1.8 w/v% of salicylic acid, and 0.05 w/v% of piroctone olamine and 0.0075 w/v% rosemary leaf extract, individually and all together in combination for 1 hour. The keratinocytes are washed in PBS and ATP levels are measured using the Cell Titer-Glo^{®} system (Promega cat# G7571/2/3, Madison, WI) per manufacturer's directions. Luminescence is measured on a SpectraMax M3 (Molecular Devices, Sunnyvale, CA). Another example would be treatment of a constant dilution of 0.05 w/v% of piroctone olamine, and 0.0075 w/v% of rosemary leaf extract, and 0.1 v/v% of glycerin individually and in combination for one hour. Net luminescence is calculated by subtracting the luminescence counts from the vehicle control from the luminescence counts from the treatment groups. Synergy is defined by the observed luminescence value from the combination exceeding (with p-value <0.05) the sum of luminescence values of each individual component.

Student's T-Test (equal variance, 2 sided) are used to calculate p-values between observed combinations and expected combinations with p-value < 0.05 considered statistically significant. Expected combination values are calculated by adding the net luminescence values of each material tested alone. Synergy factor is the ratio of observed combination/expected combination. Synergy factors greater than 1.0 with a p-value <0.05 meet the definition of synergism.

### RESULTS

### Summary of ATP Levels Post-Peroxide Exposure

The tables below are a summary of the synergy data for rosemary + piroctone olamine, rosemary + piroctone olamine + salicylic acid and rosemary + piroctone olamine + glycerin in the Peroxide-Induced Keratinocyte Stress assay measuring HOOH-ATP activity (peroxide ATP activity). The p-value < 0.05 observed vs calculated for rosemary + piroctone olamine, rosemary + piroctone olamine + salicylic acid and rosemary + piroctone olamine + glycerin is demonstrated below in the bolded values demonstrating statistically, significant synergies between rosemary + piroctone olamine, rosemary + piroctone olamine + salicylic acid and rosemary + piroctone olamine + glycerin. Synergy is most easily visualized by the SYNERGY FACTOR column which is the observed value from the combination divided by the sum of each component individually. Perfect additivity can result in a synergy factor of 1.00 so values above 1 display synergy and values above 1 AND have p-value <0.05 between observed and expected combinations meet the synergy as demonstrated in the present disclosure and as demonstrated in the highlighted bold boxes in the table below.

For combinations of rosemary + piroctone olamine, synergy exists with a minimum level of 0.001% or greater of rosemary leaf extract and 0.0025% or greater for piroctone olamine demonstrating antioxidant synergy. Therefore, a ratio greater than 1:100 between rosemary leaf extract and piroctone olamine, will demonstrate antioxidant synergy.

For combinations of rosemary + piroctone olamine + salicylic acid, synergy exists with a minimum level of 0.0075% or greater for rosemary and 0.0025% or greater for piroctone olamine and 0.5% or greater for salicylic acid demonstrating antioxidant synergy.

For combinations of rosemary + piroctone olamine + glycerin, synergy exists with a minimum level of 0.0075 or greater for rosemary and 0.0025 or greater for piroctone olamine and 0.001% or greater for glycerin demonstrating antioxidant synergy.

With the exception the 2 samples below that have piroctone olamine and rosemary leaf extract at very low concentrations (piroctone olamine 0.0025 w/v% with 0.001w/v% rosemary leaf extract and piroctone olamine 0.0025 w/v% with 0.0001 w/v% rosemary leaf extract ), as long as a ratio is greater than 1:100 between rosemary leaf extract and piroctone olamine, there will be synergy.

| **PO Level (w/v% )** | **Rosemar y Extract Level (w/v%)** | **PO: Rosemar y Extract Ratio** | **Experimental HOOH-ATP Assay Net Luminescenc e** | **Predicted HOOH-ATP Assay Net Luminescenc e** | **Synergy Factor Experimental/Predicte d** |
|---|---|---|---|---|---|
| 0.05 | 0 | | 33059 | | |
| 0.01 | 0 | | 1451 | | |
| 0.0025 | 0 | | 363 | | |
| 0 | 0.01 | | 402 | | |
| 0 | 0.005 | | 429 | | |
| 0 | 0.001 | | 210 | | |
| 0 | 0.0001 | | 435 | | |
| 0.05 | 0.01 | 1:5 | 74329 | 33462 | **2.22** |
| 0.05 | 0.005 | 1:10 | 44713 | 33489 | **1.34** |
| 0.05 | 0.001 | 1:50 | 39210 | 33270 | **1.18** |
| 0.05 | 0.0001 | 1:500 | 36652 | 33495 | 1.09 |
| 0.01 | 0.01 | 1:1 | 9389 | 1853 | **5.07** |
| 0.01 | 0.005 | 1:2 | 4746 | 1880 | **2.52** |
| 0.01 | 0.001 | 1:10 | 2253 | 1661 | **1.36** |
| 0.01 | 0.0001 | 1:100 | 1989 | 1886 | 1.05 |
| 0.0025 | 0.01 | 4:1 | 1034 | 765 | **1.35** |
| 0.0025 | 0.005 | 2:1 | 1645 | 792 | **2.08** |
| 0.0025 | 0.001 | 2:5 | 612 | 573 | 1.07 |
| 0.0025 | 0.0001 | 1:25 | 667 | 798 | 0.84 |
| 0.5 | 0.0075 | 1:66 | 4237 | 6609 | **1.56** |

| **PO Level (w/v %)** | **Rosema ry Extract Level (w/v%)** | **Glycer in level (v/v%)** | **PO: Rosemary Extract: glycerin Ratio** | **Experimen tal HOOH-ATP Assay Net Luminesce nce** | **Predicted HOOH-ATP Assay Net Luminesce nce** | **Synergy Factor experimental/predi cted** |
|---|---|---|---|---|---|---|
| 0.05 | 0.0075 | 0.1 | 1:0.15:2 | 1467 | 1012 | **1.45** |
| 0.05 | 0.0075 | 0.05 | 1:0.15:1 | 1179 | 892 | **1.32** |
| 0.05 | 0.0075 | 0.01 | 1:0.15:0.2 | 956 | 818 | **1.17** |
| 0.05 | 0.0075 | 0.005 | 1:0.15:0.1 | 811 | 805 | 1.01 |

| **PO Level (w/v %)** | **Rosema ry Extract Level (w/v%)** | **Salicyl ic Acid level (v/v% )** | **PO: Rosemary Extract:Salic ylic Acid Ratio** | **Experimen tal HOOH-ATP Assay Net Luminesce nce** | **Predicted HOOH-ATP Assay Net Luminesce nce** | **Synergy Factor experimental/pred icted** |
|---|---|---|---|---|---|---|
| 0.05 | 0.0075 | 1.8 | 1:0.15:36 | 1467 | 888 | **1.65** |
| 0.05 | 0.0075 | 1 | 1:0.15:20 | 1203 | 864 | **1.39** |
| 0.05 | 0.0075 | 0.5 | 1:0.15:10 | 987 | 834 | **1.18** |
| 0.05 | 0.0075 | 0.1 | 1:0.15:2 | 831 | 805 | 1.03 |

### Non-limiting Examples

The personal care compositions illustrated in the following examples are prepared by conventional formulation and mixing methods. All exemplified amounts are listed as weight percents on an active basis and exclude minor materials such as diluents, preservatives, color solutions, imagery ingredients, botanicals, and so forth, unless otherwise specified. All percentages are based on weight unless otherwise specified.

### Shampoo Examples

| Example | 1 | 2 | 3 | 4 | 5 |
|---|---|---|---|---|---|
| Ingredients | Active wt % | | | | |
| Sodium Laureth-1 Sulfate ¹ | 6 | 8 | 11 | 13 | 13 |
| Sodium Deceth-1 Sulfate ² | 2 | 1 | 3 | | |
| Cocamidopropyl Betaine ³ | | 1 | | 1 | 1 |
| Cocamide MEA ⁴ | | | | 0.25 | 0.2 |
| Piroctone Olamine ⁵ | 0.5 | 0.2 | 0.5 | 0.8 | 1.0 |
| Zinc Pyrithione⁶ | - | 1 | | | 1 |
| Glycerin⁷ | - | 0.5 | 1 | 5 | 10 |
| Rosemary Leaf Extract ⁸ | 0.0075 | 0.0075 | 0.0075 | 1.8 | 1.8 |
| Guar Hydroxypropyltrimonium Chloride ⁹ | | 0.5 | | | |
| Guar Hydroxypropyltrimonium Chloride ¹⁰ | | | 0.2 | | |
| Guar Hydroxypropyltrimonium Chloride¹¹ | | | | 0.4 | 0.4 |
| Polyquaternium-76 ¹² | 0.25 | | | | |
| Polyquatemium-10 ¹³ | | | 0.45 | | |
| Acrylates Copolymer ¹⁴ | 2.5 | 0.9 | | 0.7 | 0.7 |
| Sodium Benzoate ¹⁵ | 0.25 | 0.25 | 0.25 | 0.25 | 0.25 |
| Tetrasodium EDTA ¹⁶ | 0.13 | 0.13 | 0.13 | 0.13 | 0.13 |
| Methylchloroisothiazolinone/Methylisothiazolinone ¹⁷ | 5 ppm | | 5 ppm | | |
| Salicylic Acid ¹⁸ | | - | 0.8 | | 3.0 |
| Citric Acid ¹⁹ | Up to 2 | Up to 2 | Up to 2 | Up to 2 | Up to 2 |
| Sodium Citrate ²⁰ | Up to 2 | Up to 2 | Up to 2 | Up to 2 | Up to 2 |
| Fragrance | Up to 2 | Up to 2 | Up to 2 | Up to 2 | Up to 2 |
| Sodium Chloride ²¹ | Up to 3 | Up to 3 | Up to 3 | Up to 3 | Up to 2 |
| Water | q.s. | q.s. | q.s. | q.s. | q.s |
| pH | 5.3 | 4.3 | 5 | 5.3 | 5.8 |
| key | | | | | |
| ¹ Sodium Laureth-1 Sulfate at 26% active, supplier P&G | | | | | |
| ² Sodium Deceth-1 Sulfate at 34% active, supplier P&G | | | | | |
| ³ Tego Betain L 7 OK at 30% active, supplier: Evonik | | | | | |
| ⁴ Ninol Comf at 85% active, supplier: Stepan | | | | | |
| ⁵ Octopirox, supplier: Clariant | | | | | |
| ⁶ ZPT, supplier: Arch Chemical | | | | | |
| ⁷ Glycerin, supplier: Cargill, Inc. | | | | | |
| ⁸ Rosemary Leaf Extract, supplier: Symrise | | | | | |
| ⁹ N-Hance BF-13: MW = 800,000 g/mol, CD = 1.1 meq/g, supplier: Ashland Specialty Ingredients | | | | | |
| ¹⁰ Jaguar Excel: MW = 1,500,000 g/mol, CD = 0.7 meq/g, supplier Solvay | | | | | |
| ¹¹ N-Hance ^{™} 3196, Ashland | | | | | |
| ¹² Mirapol AT-1 at 10% active: MW = 1,100,000 g/mol, CD = 1.6 meq/g, supplier: Solvay | | | | | |
| ¹³ UCARE Polymer JR-30M: MW = 2,000,000 g/mol, CD = 1.3 meq/g, supplier: Dow Chemical | | | | | |
| ¹⁴ Carbopol Aqua SF-1 at 30% active, supplier: Lubrizol | | | | | |
| ¹⁵ Sodium Benzoate Dense NF/FCC, supplier: Emerald Performance Materials | | | | | |
| ¹⁶ Dissolvine 220-S at 84% active, supplier: Akzo Nobel | | | | | |
| ¹⁷ Kathon CG at 1.5% active, supplier: Rohm & Haas | | | | | |
| ¹⁸ Salicylic Acid, USP, supplier: Novacyl | | | | | |
| ¹⁹ Citric Acid Anhydrous, supplier: Archer Daniels Midland; level adjustable to achieve target pH | | | | | |
| ²⁰ Sodium Citrate Dihydrate, supplier: ADM Company; level adjustable to achieve target pH | | | | | |
| ²¹ Sodium Chloride, supplier: Morton; level adjustable to achieve target viscosity | | | | | |

### Shampoo Examples

| Example compositions | 6 | 7 | 8 | 9 | 10 |
|---|---|---|---|---|---|
| Ingredients | active wt % | | | | |
| Sodium Cocoyl isethionate (1) | | | | 6 | 6 |
| Sodium Lauroyl methyl isethionate (2) | 6 | 6 | 6 | | |
| Sodium Lauroyl Sarcosinate (3) | 4 | 2 | | 4 | 4 |
| Lauramidopropyl Betaine (4) | 2 | 4 | 6 | | |
| Cocamidopropyl Betaine (5) | | | | 6 | 4 |
| Acrylate copolymer (6) | 0.7 | 0.7 | 0.7 | 0.7 | 0.7 |
| Glycol Distearate (7) | | 0.2 | | 0.2 | |
| Piroctone Olamine (8) | 0.5 | 0.2 | 0.5 | 0.8 | 1.0 |
| Zinc Pyrithione (9) | | 1 | | 1 | 1 |
| Glycerin (10) | | 0.5 | 1 | 5 | 10 |
| Rosemary Leaf Extract (11) | 0.0075 | 0.0075 | 0.0075 | 1.8 | 1.8 |
| Salicylic Acid (12) | 0 | 0 | 1.8 | 0 | 3.0 |
| Polyquaternium-10 (13) | 0.6 | 0.6 | 0.6 | 0.6 | 0.25 |
| Guar Hyrdroxypropyltrimonium Chloride (LMW)(14) | 0.2 | | 0.4 | | 0.2 |
| Polyquatemium-76 (15) | | 0.2 | | 0.2 | |
| Perfume (16) | 1 | 1 | 1 | 1 | 1 |
| Dimethicone DC 1872 (17) | | 0.5 | | 0.5 | 1 |
| Dimethicone DC330M (18) | 1 | | 0.5 | | |
| Citric acid (19) | QS to pH 5 | QS to pH 5 | QS to pH 5.8 | QS to pH 5.8 | QS to pH 5.8 |
| Sodium Salicylate (20) | 0.15 | 0.15 | 0.15 | 0.15 | 0.15 |
| Sodium Chloride (21) | Up to 3 | Up to 3 | Up to 3 | Up to 3 | Up to 3 |
| Sodium Benzoate (22) | 0.25 | 0.25 | 0.25 | 0.25 | 0.25 |
| Water and Minors (QS to 100%) (23) | QS | QS | QS | QS | QS |
| Key | | | | | |
| 1 Sodium Cocoyl isethionate from Innospec | | | | | |
| 2 Sodium Lauroyl methyl isethionate from Innospec | | | | | |
| 3 Sodium Lauroyl Sarcosinate from Croda | | | | | |
| 4 Lauramidopropyl Betaine less than 1% NaCl from Solvay | | | | | |
| 5 Lauramidopropyl Betaine less than 1% NaCl from Evonik | | | | | |
| 6 Acrylate copolymer from BASF | | | | | |
| 7 Glycol Distearate from Goldschmidt Chemical Company | | | | | |
| 8 Octopirox from Clariant | | | | | |
| 9 ZPT from Arch Chemical | | | | | |
| 10 Glycerin from Cargill, Inc. | | | | | |
| 11 Rosemary Leaf Extract from Symrise | | | | | |
| 12 Salicylic Acid, USP from Novacyl | | | | | |
| 13 Polyquaternium-10 from Dow | | | | | |
| 14 Guar Hyrdroxypropyltrimonium Chloride (LMW) ~500k MW, 0.7 CD from Ashland | | | | | |
| 15 Polyquaternium-76 from Solvay | | | | | |
| 16 Perfume from Givuadan | | | | | |
| 17 Dimethicone DC 1872 from Dow | | | | | |
| 18 Dimethicone DC330M from Dow | | | | | |
| 19 Citric acid from Acher Daniels Midland | | | | | |
| 20 Sodium Salicylate from Novacyl | | | | | |
| 21 Sodium Chloride from Morton | | | | | |
| 22 Sodium Benzoate from Kalama Chemical | | | | | |
| 23 Water and Minors (QS to 100%) from Misty Mountain Spring water | | | | | |

### Leave On Treatment Examples

| Examples | 11 | 12 | 13 | 14 |
|---|---|---|---|---|
| Ingredients | Active wt %) | | | |
| Water | Q.S. | Q.S. | Q.S | Q.S. |
| Alcohol 100% (Ethanol) | 50 | 50 | 50 | 50 |
| Acrylates/C10-30 alkyl acrylate crosspolymer *1 | 0.35 | | | |
| FlexiThix ^{™} *2 | | 2.60% | | |
| Hydroxypropylmethylcellulose *3 | | | 0.6 | 0.6 |
| PVP K90 *4 | | | 0.4 | 0.4 |
| Bis-PEG/PPG-16/16 Dimethicone *5 | 0.7 | | | |
| Panthenol *6 | 0.15 | 0.2 | 0.2 | 0.2 |
| Niacinamide *7 | 2.5 | 2.5 | 1.9 | 2.5 |
| Caffeine *8 | 0.75 | | | 0.75 |
| Piroctone Olamine*9 | 0.005 | 0.2 | 0.45 | 0.45 |
| Zinc Pyrithione *10 | 0.1 | 0.1 | | 0.1 |
| Glycerin *11 | 0.1 | | | 5 |
| Rosemary Leaf Extract *12 | 0.0075 | 0.17 | 1.7 | 0.0075 |
| Salicylic Acid *13 | | 0.5 | | 1.8 |
| Butylene Glycol *14 | 1 | | | |
| Benzyl Alcohol *15 | 0.5 | | | |
| Tetrahydroxypropyl Ethylenediamine *16 | 0.05 | | | |
| Citric Acid*17 | Qs to pH 5.0 | Qs to pH 6 | QS to pH 5.0 | QS to pH 6 |
| Perfume | Up to 1% | Up to 1% | Up to 1% | Up to 1% |
| Key | | | | |
| *1 as in Carbopol Ultrez 21 available from Lubrizol | | | | |
| *2 as in FlexiThix ^{™} from Ashland Chemical. | | | | |
| *3 as in Cellosize 40-0101HF^{™} from Ashland Chemical | | | | |
| *4 as in PVP (K-90 Powder) from ISP Chemicals LLC | | | | |
| *5 as in Abil Care 85 from Evonik | | | | |
| *6 as in Dexapaanthenol USP from Roche Vitamins | | | | |
| *7 as in Niacinamide USP from Roche Vitamins | | | | |
| *8 as in Caffeine USP from BASF | | | | |
| *9as Octopirox from Clariant | | | | |
| *10 ZPT from Arch Chemical | | | | |
| *11 Glycerin from Cargill, Inc. | | | | |
| *12 as in Rosemary Leaf Extract from Symrise | | | | |
| *13 Salicylic Acid, USP, from Novacyl | | | | |
| *14 as in Cosmetic Quality 1,3-Butylene Glycol from Oxea Corporation | | | | |
| *15 as in Benzyl Alcohol from Polarome International | | | | |
| *16as in Neutrol Te from BASF | | | | |
| *17 as in Citric acid from Acher Daniels Midland | | | | |

### Conditioner Examples

| Components | 15 | 16 | 17 | 18 | 19 |
|---|---|---|---|---|---|
| Piroctone Olamine*1 | 0.005 | 0.8 | 0.5 | 0.8 | 0.5 |
| Zinc Pyrithione *2 | | 0.5 | | 0.5 | 0.1 |
| Glycerin *3 | | | 0.5 | 5 | 10 |
| Rosemary Leaf Extract *4 | 0.0075 | 0.0075 | 0.0075 | 1.8 | 1.8 |
| Salicylic Acid *5 | | 0.5 | | 0.5 | |
| Behenyl trimethyl ammonium chloride *6 | | | | 2.5 | 0.9 |
| Behenyl trimethyl ammonium methosulfate *7 | 2.6 | 2.6 | 2.97 | | |
| Dicetyl dimethyl ammonium chloride *8 | | 0.35 | | | |
| Cetyl alcohol *9 | 1 | 1 | 1.18 | 1 | 1 |
| Stearyl alcohol *10 | 2.4 | 2.4 | 2.95 | 2.3 | 2.3 |
| Aminosilicone *11 | 0.5 | 0.5 | 1.5 | 0.5 | 0.5 |
| Benzyl alcohol *12 | | | 0.4 | | |
| Preservatives | 0.9 | 0.9 | 0.9 | 0.9 | 0.9 |
| Perfume | 0.5 | 0.5 | 0.5 | 0.5 | 0.5 |
| Deionized Water q.s. to 100% | q.s | q.s | q.s | q.s | q.s |

| Definitions of Components | | | | | |
|---|---|---|---|---|---|
| KEY | | | | | |
| *1 Octopirox from Clariant | | | | | |
| *2 ZPT from Arch Chemical | | | | | |
| *3 Glycerin from Cargill, Inc. | | | | | |
| *4 Rosemary Leaf Extract from Symrise | | | | | |
| *5 Salicylic Acid, USP, from: Novacyl | | | | | |
| *6 Clariant | | | | | |
| *7 Clariant | | | | | |
| *8 Clariant | | | | | |
| *9 BASF | | | | | |
| *10 Cognis | | | | | |
| *11 Aminosilicone: Terminal aminosilicone which is available from GE having a viscosity of about 10,000mPa s, and having following formula: (R1)aG3-a-Si-(-OSiG2)n-O-SiG3-a(R1)a wherein G is methyl; a is an integer of 1; n is a number from 400 to about 600; R1 is a monovalent radical conforming to the general formula CqH2qL, wherein q is an integer of 3 *12 Emerald Kalama Chemical | | | | | |

### PRODUCT FORM

The personal care compositions may be presented in typical personal care formulations. They may be in the form of solutions, dispersion, emulsions, powders, talcs, encapsulated, spheres, spongers, solid dosage forms, foams, and other delivery mechanisms. The personal care compositions may be hair tonics, leave-on hair products such as treatment, and styling products, rinse-off hair products such as shampoos, pre-wash product, co-wash product, and personal cleansing products, and treatment products; and any other form that may be applied to hair or scalp.

### Additional Examples/Combinations

A. A personal care composition comprising: a) rosemary leaf extract; b) a 2-pyridinol-N-oxide material wherein at a level of 0.001% or greater of a and at a level of 0.0025% or greater of b there is a synergistic anti-antioxidant/cellular energetic activity.
B. A personal care composition according to Paragraph A, comprising: a) rosemary leaf extract; b) a 2-pyridinol-N-oxide material; and c) salicylic acid wherein at a level of 0.0075% or greater of a and at a level of 0.0025% or greater of b and at a level of 0.5% or greater of c there is a synergistic anti-antioxidant/cellular energetic activity.
C. A personal care composition according to Paragraph A-B, comprising a) rosemary leaf extract; b) a 2-pyridinol-N-oxide material; and c) glycerin wherein at a level of 0.0075% or greater of a and at a level of 0.0025% or greater of b and at a level of 0.001% or greater of c there is a synergistic anti-antioxidant/cellular energetic activity.
D. A personal care composition according to Paragraph A-C, wherein the anti-oxidant benefit is measured by peroxide ATP activity.
E. A personal care composition according to Paragraph A-D, wherein the anti-oxidant benefit is measured by peroxide ATP activity.
F. A personal care composition according to Paragraph A-E, wherein the anti-oxidant benefit is measured by peroxide ATP activity.
G. A personal care composition according to Paragraph A-F, wherein the hair and/or scalp care composition is selected from group consisting of a shampoo, conditioner, leave-on, tonic and mixtures thereof.
H. A personal care composition according to Paragraph A-G, wherein the hair and/or scalp care composition is selected from group consisting of a shampoo, conditioner, leave-on, tonic and mixtures thereof.
I. A personal care composition according to Paragraph A-H, wherein the hair and/or scalp care composition is selected from group consisting of a shampoo, conditioner, leave-on, tonic and mixtures thereof.
J. A personal care composition according to Paragraph A-I, wherein the rosemary leaf extract is from about 0.001% to about 3%.
K. A personal care composition according to Paragraph A-J, wherein the 2-pyridinol-N-oxide material is from about 0.00001% to about 5%, by weight of the hair and/or scalp care composition.
L. A personal care composition according to Paragraph A-K, wherein the 2-pyridinol-N-oxide material is selected from the group consisting of 1-Hydroxy-4-methyl-6-(2,4,4-trimethylpentyl)-2-pyridone monoethanolamine salt, 6-Cyclohexyl-1-hydroxy-4-methyl-2(1H)-pyridone ethanolammonium salt, 6-[[p-chlorophenoxy )phenoxy]methyl]-1 hydroxy-4-methyl--pyridone.
M. A personal care composition according to Paragraph A-I, wherein the 2-pyridinol-N-oxide material is piroctone olamine.
N. A personal care composition according to Paragraph A-M, wherein the rosemary leaf extract is from about 0.001% to about 3%.
O. A personal care composition according to Paragraph A-N, wherein the glycerin is from about 0.05% to about 30%.
P. A personal care composition according to Paragraph A-O, wherein the 2-pyridinol-N-oxide material is from about 0.00001% to about 5%, by weight of the hair and/or scalp care composition.
Q. A personal care composition according to Paragraph A-P, wherein the 2-pyridinol-N-oxide material is selected from the group consisting of 1-Hydroxy-4-methyl-6-(2,4,4-trimethylpentyl)-2-pyridone monoethanolamine salt, 6-Cyclohexyl-1-hydroxy-4-methyl-2(1H)-pyridone ethanolammonium salt, 6-[[p-chlorophenoxy )phenoxy]methyl]-1 hydroxy-4-methyl--pyridone.
R. A personal care composition according to Paragraph A-Q, wherein the 2-pyridinol-N-oxide material is piroctone olamine.
S. A personal care composition according to Paragraph A-R, wherein a ratio greater than 1:100 between (a) rosemary leaf extract and (b) piroctone olamine provides a synergistic anti-antioxidant/cellular energetic activity.
T. A personal care composition according to Paragraph A-S, wherein a method of using a personal care composition comprising: a) rosemary leaf extract; b) a 2-pyridinol-N-oxide material wherein at a level of 0.001% or greater of a and at a level of 0.0025% or greater of b the personal care composition provides a synergistic anti-antioxidant/cellular energetic activity.
U. A personal care composition according to Paragraph A-T, wherein a method of using a personal care composition comprising: a) rosemary leaf extract; b) a 2-pyridinol-N-oxide material; and c) salicylic acid wherein at a level of 0.0075% or greater of a and at a level of 0.0025% or greater of b and at a level of 0.5% or greater of c the personal care composition provides is a synergistic anti-antioxidant/cellular energetic activity.
V. A personal care composition according to Paragraph A-U, wherein A method of using a personal care composition comprising: a) rosemary leaf extract; b) a 2-pyridinol-N-oxide material; and c) glycerin wherein at a level of 0.0075% or greater of a and at a level of 0.0025% or greater of b and at a level of 0.001% or greater of c the personal care composition provides is a synergistic anti-antioxidant/cellular energetic activity.
W. A personal care composition comprising: a) rosemary leaf extract in an amount of from about 0.001% to about 3% by weight of the composition; and b) piroctone olamine in an amount of from about 0.0025% to about 5% by weight of the composition; wherein the personal care composition provides a synergistic antioxidant/cellular energetic activity.
X. A personal care composition comprising: a) rosemary leaf extract; and b) piroctone olamine; wherein the rosemary leaf extract is present at a level of 0.001% by weight of the composition or greater, the piroctone olamine is present at a level of 0.0025% by weight of the composition or greater, and the weight ratio of rosemary leaf extract to piroctone olamine is greater than 1:100, such that the personal care composition provides a synergistic antioxidant/cellular energetic activity.
Y. A personal care composition comprising: a) rosemary leaf extract in an amount of from about 0.001% to about 3% by weight of the composition; b) piroctone olamine in an amount of from about 0.0025% to about 5% by weight of the composition; and c) salicylic acid in an amount of from about 0.1% to about 10% by weight of the composition; wherein the rosemary leaf extract is present at a level of 0.0075% by weight of the composition or greater, the piroctone olamine is present at a level of 0.0025% by weight of the composition or greater, and the salicylic acid is present at a level of 0.5% by weight of the composition or greater, such that the personal care composition provides a synergistic antioxidant/cellular energetic activity.
Z. A personal care composition comprising: a) rosemary leaf extract in an amount of from about 0.001% to about 3% by weight of the composition; b) piroctone olamine in an amount of from about 0.0025% to about 5% by weight of the composition; and c) glycerin in an amount of from about 0.05% to about 30% by weight of the composition; wherein the rosemary leaf extract is present at a level of 0.0075% by weight of the composition or greater, the piroctone olamine is present at a level of 0.0025% by weight of the composition or greater, and the glycerin is present at a level of 0.001% by weight of the composition or greater, such that the personal care composition provides a synergistic antioxidant/cellular energetic activity.

AA. A personal care composition comprising: a) rosemary leaf extract in an amount of from about 0.001% to about 1% by weight of the composition; and b) piroctone olamine in an amount of from about 0.5% to about 2% by weight of the composition; wherein the personal care composition provides a synergistic antioxidant/cellular energetic activity.
AB. A personal care composition comprising: a) rosemary leaf extract in an amount of from about 0.0075% to about 1% by weight of the composition; b) piroctone olamine in an amount of from about 0.5% to about 2% by weight of the composition; and c) salicylic acid in an amount of from about 0.5% to about 2% by weight of the composition; wherein the personal care composition provides a synergistic antioxidant/cellular energetic activity.
AC. A personal care composition comprising: a) rosemary leaf extract in an amount of from about 0.0075% to about 1% by weight of the composition; b) piroctone olamine in an amount of from about 0.5% to about 2% by weight of the composition; and c) glycerin in an amount of from about 0.5% to about 10% by weight of the composition; wherein the personal care composition provides a synergistic antioxidant/cellular energetic activity.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to anything disclosed or claimed herein or that it alone, or in any combination with any other reference or references, teaches, suggests or discloses anything disclosed or claimed herein. Further, to the extent that any meaning or definition of a term in this document conflicts with any meaning or definition of the same term in a document incorporated by reference, the meaning or definition assigned to that term in this document shall govern.

While particular forms of the present disclosure have been illustrated and described, it would be obvious to those skilled in the art that various other changes and modifications can be made without departing from the spirit and scope of the present disclosure. It is therefore intended to cover in the appended claims all such changes and modifications that are within the scope of the present disclosure.

## Claims

1. A personal care composition comprising:
a) rosemary leaf extract;
b) a 2-pyridinol-N-oxide material
wherein at a level of 0.001% by weight of the composition or greater of a and at a level of 0.0025% by weight of the composition or greater of b there is a synergistic anti-antioxidant/cellular energetic activity.

2. A personal care composition according to claim 1, wherein the composition further comprises:
c1) salicylic acid
wherein at a level of 0.0075% by weight of the composition or greater of a and at a level of 0.0025% by weight of the composition or greater of b and at a level of 0.5% by weight of the composition or greater of c1 there is a synergistic anti-antioxidant/cellular energetic activity.

3. A personal care composition according to claim 2 wherein the salicylic acid is from 0.1% to 10% by weight of the composition.

4. A personal care composition according to any one of the preceding claims wherein the composition further comprises:
c2) glycerin
wherein at a level of 0.0075% by weight of the composition or greater of a and at a level of 0.0025% by weight of the composition or greater of b and at a level of 0.001% by weight of the composition or greater of c2 there is a synergistic anti-antioxidant/cellular energetic activity.

5. A personal care composition according to claim 4 wherein the glycerin is from 0.05% to 30% by weight of the composition.

6. A personal care composition according to any one of the preceding claims wherein the anti-oxidant benefit is measured by peroxide ATP activity,
optionally wherein the anti-oxidant benefit is measured according to the Peroxide-Induced Keratinocyte Stress Method defined herein.

7. A personal care composition according to any one of the preceding claims wherein the hair and/or scalp care composition is selected from group consisting of a shampoo, conditioner, leave-on, tonic and mixtures thereof.

8. A personal care composition according to any one of the preceding claims wherein the rosemary leaf extract is from 0.001% to 3% by weight of the composition.

9. A personal care composition according to any one of the preceding claims wherein the 2-pyridinol-N-oxide material is from 0.0025% to 5%, by weight of the personal care composition.

10. A personal care composition according to any one of the preceding claims wherein the 2-pyridinol-N-oxide material is selected from the group consisting of 1-Hydroxy-4-methyl-6-(2,4,4-trimethylpentyl)-2-pyridone monoethanolamine salt, 6-Cyclohexyl-1-hydroxy-4-methyl-2(1H)-pyridone ethanolammonium salt, 6-[[p-chlorophenoxy )phenoxy]methyl]-1 hydroxy-4-methyl--pyridone.

11. A personal care composition according to any one of the preceding claims wherein the 2-pyridinol-N-oxide material is piroctone olamine.

12. A personal care composition according to any one of the preceding claims, wherein a ratio greater than 1:100 between (a) rosemary leaf extract and (b) piroctone olamine provides a synergistic anti-antioxidant/cellular energetic activity.

13. A method of using a personal care composition as defined in any one of the preceding claims comprising:
a) rosemary leaf extract;
b) a 2-pyridinol-N-oxide material
wherein at a level of 0.001% by weight of the composition or greater of a and at a level of 0.0025% by weight of the composition or greater of b the personal care composition provides a synergistic anti-antioxidant/cellular energetic activity.

14. A method of using a personal care composition according to any preceding claims comprising:
a) rosemary leaf extract;
b) a 2-pyridinol-N-oxide material; and further comprising
c) salicylic acid
wherein at a level of 0.0075% by weight of the composition or greater of a and at a level of 0.0025% by weight of the composition or greater of b and at a level of 0.5% by weight of the composition or greater of c the personal care composition provides a synergistic anti-antioxidant/cellular energetic activity.

15. A method of using a personal care composition according to any preceding claims comprising:
a) rosemary leaf extract;
b) a 2-pyridinol-N-oxide material; and further comprising
c) glycerin
wherein at a level of 0.0075% by weight of the composition or greater of a and at a level of 0.0025% by weight of the composition or greater of b and at a level of 0.001% by weight of the composition or greater of c the personal care composition provides a synergistic anti-antioxidant/cellular energetic activity.
